# EUROPEAN PATENT SPECIFICATION

(11) **EP 2 817 305 B1**
(45) Date of publication and mention of the grant of the patent: **28.09.2016**
(21) Application number: 13752547.3
(22) Date of filing: 22.02.2013
(51) Int. Cl.: C07D 413/06, C07D 265/32, A61K 31/5377

(54) **AN IMPROVED PROCESS FOR THE PREPARATION OF APREPITANT**
VERBESSERTES VERFAHREN ZUR ZUBEREITUNG VON APREPITANT
PROCÉDÉ PERFECTIONNÉ POUR LA PRÉPARATION D'APRÉPITANT

(30) Priority: 23.02.2012 IN MM04952012
(43) Date of publication of application: 31.12.2014
(73) Proprietor: Piramal Enterprises Limited, Mumbai 400 013 (IN)
(72) Inventor: LADKAT, Prashant, Mumbai 400063 (IN); WAGH, Ganesh, Mumbai 400063 (IN); JAGTAP, Ashutosh, Mumbai 400063 (IN); ROY, Mita, Mumbai 400063 (IN); HARIHARAN, Sivaramakrishnan, Mumbai 400063 (IN); CHOUKEKAR, Milind, Mumbai 400063 (IN)
(74) Representative: Richards, Michèle E.
(86) International application number: PCT/IB2013/051440
(87) International publication number: WO 2013/124823

(56) References cited:
- WO-A1-03/089429
- WO-A1-2011/147279
- WO-A2-2007/039883
- KAREL M J BRANDS ET AL: "Efficient Synthesis of NK1 Receptor Antagonist Aprepitant Using a Crystallization-Induced Diastereoselective Transformation", JOURNAL OF THE AMERICAN CHEMICAL SOCIETY, [NOT]AMERICAN CHEMICAL SOCIETY|, vol. 125, 1 January 2003 (2003-01-01), pages 2129-2135, XP002374343, ISSN: 0002-7863, DOI: 10.1021/JA027458G
- ELATI, C. R. ET AL.: 'A Convergent Approach to the Synthesis of Apreptiant: A Potent Human NK-1 Receptor Antagonist' TETRAHEDRON LETTERS vol. 48, 2007, pages 8001 - 8004, XP022293826

## Description

### FIELD OF THE INVENTION

The present invention relates to a process for the preparation of 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one (Aprepitant). More particularly, the present invention relates to an in-situ process for the preparation of aprepitant.

### BACKGROUND OF THE INVENTION

Aprepitant is chemically known as, 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one, structurally represented herein below by formula I,

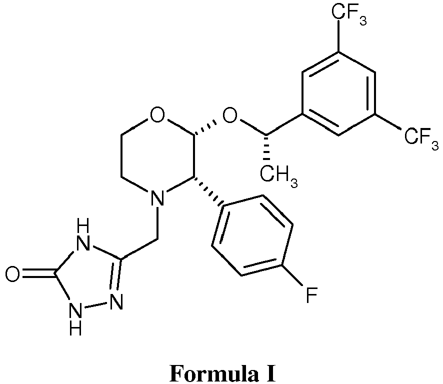

Aprepitant is an antiemetic chemical compound which belongs to the Substance P Antagonist class of drugs. Aprepitant blocks the signals given off by NK1 receptors and therefore also classified as an NK1 antagonist. NK1 is a G protein-coupled receptor and is located in the central nervous system and the peripheral nervous system. This receptor has a dominant ligand known as Substance P (SP). SP is a neuropeptide, composed of 11 amino acids, which sends and receives impulses and messages from the brain. It is found in high concentrations in the vomiting center of the brain, and, when activated, it results in a vomiting reflux occurring. Aprepitant has been shown to inhibit both the acute and delayed emesis induced by cytotoxic chemotherapeutic drugs and postoperative nausea and vomiting by blocking SP landing on receptors in the brains neurons. Aprepitant is manufactured by Merck & Co. under the brand name Emend®.

The process for the preparation of aprepitant of formula I is disclosed in the prior art documents. Generally, the process involves condensation of 2-(R)-[(1R)-1-[3,5-bis(trifluoromethyl)phenyl]ethoxy]-3-(S)-(4-fluorophenyl)morpholine of formula II or its salts with 2-(2-chloro-1-iminoethyl)hydrazinecarboxylic acid methyl ester of formula III to obtain 2-[2-[(2R,3S)-2-[(1R)-1-[3,5-bis(trifluoromethyl)phenyl]ethoxy]-3-(4-fluorophenyl)-4-morpholinyl]-1-iminoethyl]hydrazinecarboxylic acid methyl ester of formula IV, which is then cyclized to obtain aprepitant (e.g. WO2011/147279). The general process for the preparation of aprepitant disclosed in the prior art is schematically represented herein below: 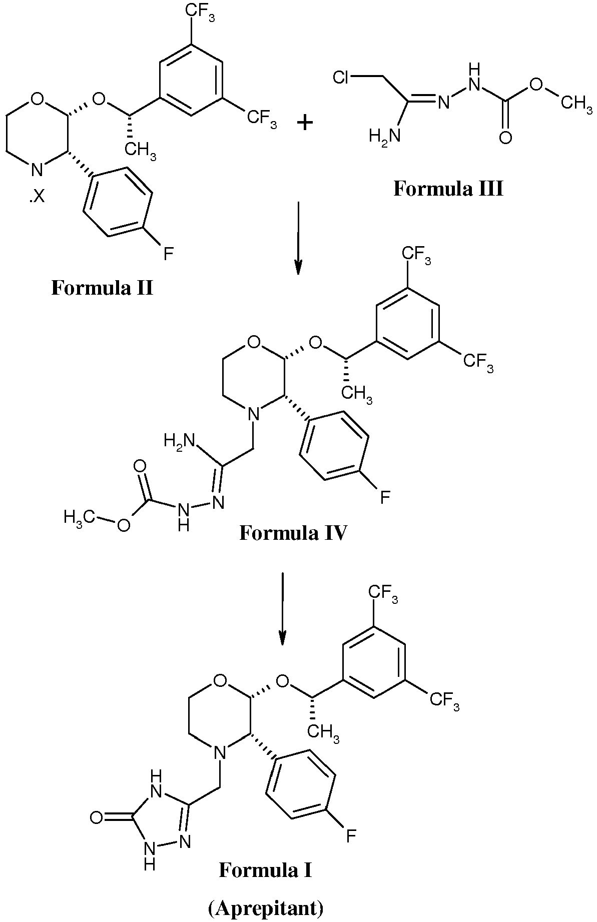 wherein in Formula II, X = H or pharmaceutically acceptable salts such as hydrochloride, oxalate, camphor sulphonate, p-toluenesulphonate, etc.

The processes for the preparation of aprepitant of formula I disclosed in the prior art documents from 2-(R)-[(1R)-1-[3,5-bis(trifluoromethyl)phenyl]ethoxy]-3-(S)-(4-fluorophenyl)morpholine of formula II or its salts are associated with several disadvantages. Mainly the processes utilize large volume of organic solvents for the condensation of compound of formula II with 2-(2-chloro-1-iminoethyl)hydrazinecarboxylic acid methyl ester of formula III to obtain 2-[2-[(2R,3S)-2-[(1R)-1-[3,5-bis(trifluoromethyl)phenyl]ethoxy]-3-(4-fluorophenyl)-4-morpholinyl]-1-iminoethyl]hydrazinecarboxylic acid methyl ester of formula IV, which is isolated from the reaction mixture as a solid, foam, viscous liquid or liquid using work-up process such as extraction, evaporation, crystallization, etc. Further, the isolated compound of formula IV is cyclized in the presence of an organic solvent at high temperature to yield aprepitant. Moreover, the isolation of compound of formula IV from the reaction mixture results in loss of yield, which eventually renders the overall yield loss in the preparation of aprepitant from the compound of formula II.

The product, Aprepitant, 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one and its pharmaceutically acceptable salts are disclosed in US Patent No. 5,719,147 (hereinafter referred to as US'147 Patent). The process for the preparation of aprepitant described in US'147 Patent comprises the steps of: (a) reaction of 2-(R)-[(1R)-1-[3,5-bis(trifluoromethyl)phenyl]ethoxy]-3-(S)-(4-fluorophenyl)morpholine (hereinafter also referred to as "free base of compound of formula II") with the compound of formula III using N,N-diisopropylethylamine as a base in the presence of acetonitrile as a solvent at a room temperature for 20 hours, the reaction mixture obtained was partitioned between methylene chloride and water and the separated organic layer was dried over magnesium sulfate and concentrated in vacuum, the resulting reaction mixture pass through flash chromatography to obtain the compound of formula IV as a foam; (b) the compound of formula IV solution in xylene was heated at a reflux temperature for 2 hours and then the resulting reaction mixture was passed through flash chromatography to yield 79% of aprepitant. The process disclosed in US'147 Patent requires isolation of compound of formula IV from the reaction mixture using extraction and evaporation methods. The process also utilizes flash chromatography to obtain the compound of formula IV and aprepitant. The step (a) of the process involves use of an organic base such as N,N-diisopropylethylamine and about 17 volumes of organic solvent, that is acetonitrile. Thus, isolation of compound of formula IV involving use of such large volume of expensive organic solvents, such as acetonitrile and use of flash chromatography techniques renders the process expensive, lengthy, tedious and industrially non-viable.

PCT Application Publication No. WO 2009/116081 (hereinafter referred to as WO'081 patent appln.) discloses a process for the preparation of aprepitant of formula I comprises the steps of: (a) treating the free base of compound of formula II with oxalic acid in methanol to precipitate oxalate salt of compound of formula II; (b) converting the resulting oxalate salt of the compound of formula II into its free base by treatment with 10% sodium hydroxide solution, which was then reacted with the compound of formula III using potassium carbonate in the presence of dimethylsulfoxide to obtain solid compound of formula IV; (c) refluxing the solution containing the compound of formula IV in xylene and N,N-diisopropylethylamine for 4-6 hours to obtain crude aprepitant; (d) purifying the crude aprepitant to obtain pure aprepitant. The process disclosed in WO'081 patent appln. is in fact a lengthy process since said process involves first converting the free base of compound of formula II to its oxalate salt and then converting the oxalate salt back into the free base of compound of formula II. Also, the process requires use of 5 volumes of organic solvent in step (a) and approximately 9 volumes of organic solvent in step (c). Therefore, such process for the preparation of aprepitant which is not only lengthy but also involves use of large volume of organic solvents thereby rendering the process industrially disadvantageous.

PCT Application Publication No. WO2009/001203 discloses a process for the preparation of aprepitant of formula I comprising the steps of: (a) treating the solution of free base of compound of formula II in methyl tert-butyl ether and heptane with (R)(-)-camphor sulphonic acid to yield camphor sulphonate salt of the compound of formula II; (b) reacting the resulting salt with the compound of formula III in the presence of potassium carbonate, dimethylformamide and a solution of N-methylcarboxy-2-chloroacetamidrazone in dimethylformamide followed by stirring till the completion of reaction. After reaction completion charged water and methyl tert-butyl ether to the reaction mixture and separated the organic layer; (c) concentrating the organic layer to obtain crude compound of formula IV; (d) dissolving the resulting compound of formula IV in xylene followed by heating to reflux at a temperature of 135°C to 140°C to yield crude aprepitant; (e) further purifying the crude aprepitant to yield pure aprepitant.

US Patent Application Publication No. 2010/0004242 (hereinafter referred to as US'242 patent appln.) discloses a process for the preparation of aprepitant of formula I involving reaction of 4-methylbenzenesulphonate salt of the compound of formula II with the compound of formula III using powdered potassium carbonate in the presence of dimethylsulfoxide at a temperature of 20°C to 23°C to obtain the reaction mixture containing the compound of formula IV, the obtained reaction mixture quenched with water and extracted with toluene. The extracted toluene layer then washed with water and concentrated the toluene layer at atmospheric pressure to the maximum extent to obtain viscous liquid residue, which was then heated at a temperature of 135°C to 137°C for 2 hours to obtain a solid residue. The solid residue on further workup yielded aprepitant. The US'242 patent appln. describing the preparation of aprepitant from the compound of formula IV reports that the cyclization of compound of formula IV was carried out in the absence of a solvent. However, the process described in said patent appln. as discussed above, in fact, describes heating of a viscous liquid, containing the compound of formula IV, at a temperature of 135°C to 137°C to obtain aprepitant. The viscous liquid used for cyclization of compound of formula IV was obtained by the concentration of toluene layer at an atmospheric pressure, which indicates the presence of toluene in the viscous liquid. Further, the cyclization of compound of formula IV was carried out at a temperature of 135°C to 137°C, which indicates that the reaction was carried out under pressure, as the boiling point of toluene is 110.6°C.

US Patent No. 7,847,095 (hereinafter referred to as US'095 Patent) discloses a process for the preparation of aprepitant of formula I comprising reacting hydrochloride salt of the compound of formula II with the compound of formula III using potassium carbonate in the presence of dimethylsulfoxide and toluene as a solvent at a temperature of 15°C, the obtained reaction mixture containing the compound of formula IV is then partitioned between toluene and water, the organic layer was separated at 40°C. The organic layer was then washed with water at 40°C and partially concentrated at atmospheric pressure providing the reaction mixture containing the compound of formula IV. The resulting reaction mixture was then heated at a temperature of 140°C for 3 hours and then allowed to cool to room temperature. The solid obtained was filtered and dried to obtain the product, aprepitant. The resulting product (aprepitant) was further dissolved in methanol and treated with darco to obtain pure aprepitant with 85% overall yield. The process disclosed in US'095 Patent involves two steps process for the preparation of aprepitant, as the process involves concentration of reaction mixture containing the compound of formula IV before cyclization of compound of formula IV. Also, the process involves work-up process involving extraction and evaporation to isolate the compound of formula IV from the reaction mixture, which is then used for the next step of cyclization. Further, the process involves carrying out the reaction at high temperature, such as 140°C for cyclization step, which renders the process industrially disadvantageous.

The process for the preparation of aprepitant of formula I can be improved particularly in terms of industrial applicability by providing cost-effective, simple and efficient process for the preparation of the product, that would also result in obtaining said product in good yield and purity. The process for the preparation of aprepitant disclosed in the cited prior art references mostly suggests two step process for the preparation of aprepitant from the compound of formula II involving condensation and cyclization, wherein the compound of formula IV is isolated from the reaction mixture after condensation reaction. The process also involves high reaction temperature and large volume of organic solvent to obtain aprepitant. Thus, there is need to develop an improved process for the preparation of aprepitant from the compound of formula II, that avoids use of large volume of organic solvent and high temperature, which is cost-effective, simple and industrially applicable.

The inventors of present invention have now found that aprepitant of formula I can be obtained in good yield and purity from 2-(R)-[(1R)-1-[3,5-bis(trifluoromethyl)phenyl]ethoxy]-3-(S)-(4-fluorophenyl)morpholine hydrochloride salt of formula II through an improved process, which involves in-situ preparation of aprepitant, avoiding isolation of compound of formula IV using any work-up processes such as extraction, evaporation, crystallization, etc. Also, the process avoids use of large volume of organic solvent for the condensation of compound of formula II with the compound of formula III and high temperature required for the cyclization of compound of formula IV to obtain aprepitant. Thus, the present invention provides a simple, cost-effective and industrially applicable process for the preparation of aprepitant, which is used for the treatment of acute and delayed chemotherapy induced nausea and vomiting and for prevention of postoperative nausea and vomiting.

### OBJECTS OF THE INVENTION

An object of the present invention is to provide an improved process for the preparation of 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one (Aprepitant) of formula I from 2-(R)-[(1R)-1-[3,5-bis(trifluoromethyl)phenyl]ethoxy]-3-(S)-(4-fluorophenyl)morpholine hydrochloride salt of formula II, wherein the compound of formula IV is cyclized in-situ to yield aprepitant, by using a mixture of dimethylsulfoxide and polar protic solvent as a reaction solvent at a temperature of 90°C to 110°C.

Another object of the present invention is to provide a process for the preparation of aprepitant with yield of ≥ 75% and purity of ≥ 99.5%.

Another object of the present invention is to provide a process for the preparation of aprepitant, which is simple, efficient, cost-effective and industrially viable.

### SUMMARY OF THE INVENTION

In accordance with the aspects of the present invention, there is provided an improved process for the preparation of 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one (Aprepitant) of formula I comprising the steps of:
(a) condensing 2-(R)-[(1R)-1-[3,5-bis(trifluoromethyl)phenyl]ethoxy]-3-(S)-(4-fluorophenyl)morpholine hydrochloride salt of formula II with 2-(2-chloro-1-iminoethyl)hydrazinecarboxylic acid methyl ester of formula III in the presence of potassium carbonate and dimethylsulfoxide to obtain a reaction mixture containing a compound of formula IV;
(b) in-situ cyclizing the reaction mixture containing compound of formula IV, obtained in step (a) to the compound of formula I by heating said reaction mixture in a mixture of dimethylsulfoxide and a polar protic solvent at a temperature of 90-110°C; and
(c) isolating aprepitant of formula I,
wherein the steps (a) and (b) are carried out without isolating intermediates from the reaction mixture.

The process of the present invention is depicted in the following scheme:

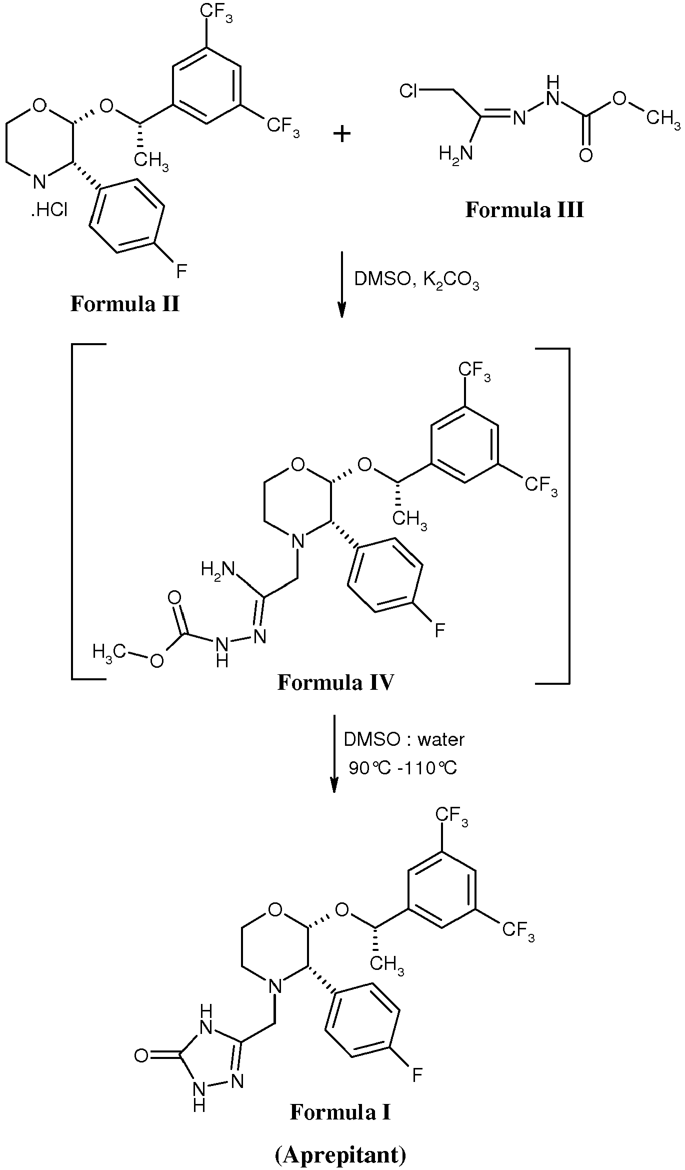

In accordance with another aspect of the present invention, the process for the preparation of aprepitant of formula I involves in-situ cyclization of the compound of formula IV to yield aprepitant, thereby avoiding the isolation of compound of formula IV in the form of solid, foam, viscous liquid, liquid, etc. using any work-up processes such as, extraction, evaporation, crystallization, etc.

In accordance with another aspect of the present invention, a process for the preparation of aprepitant of formula I, avoids isolation of compound of formula IV from the reaction mixture. Moreover, the process avoids use of any work-up processes involving extraction, evaporation, crystallization, etc to isolate the compound of formula IV in the form of solid, foam, viscous liquid, liquid, etc.

In accordance with another aspect of the present invention, aprepitant of formula I is prepared in yield ≥ 75% and purity ≥ 99.5%.

In accordance with another aspect of the present invention, the process of the present invention overcomes the disadvantages associated with the process disclosed in the cited prior arts, which mainly involves isolation of the compound of formula IV from the reaction mixture. The isolation process involves extraction, evaporation, crystallization of reaction mixture to yield the compound of formula IV in the form of solid, foam, viscous liquid, liquid, etc. Also, the process of the present invention avoids use of large volumes of organic solvent and high reaction temperature, which renders the process industrially viable.

### DETAIL DESCRIPTION OF THE INVENTION

The present invention relates to a process for the preparation of 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one (Aprepitant) of formula I, 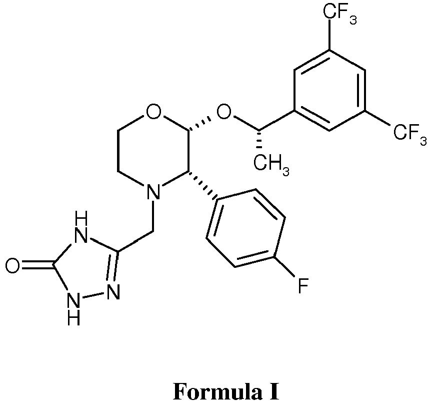 comprising the steps of:
(a) condensing 2-(R)-[(1R)-1-[3,5-bis(trifluoromethyl)phenyl]ethoxy]-3-(S)-(4-fluorophenyl)morpholine hydrochloride salt of formula II, 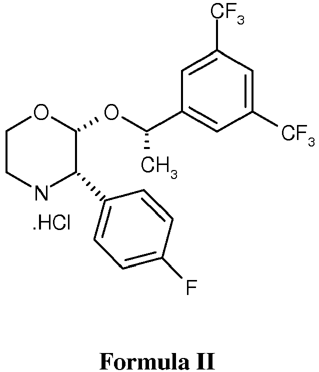 with 2-(2-chloro-1-iminoethyl)hydrazinecarboxylic acid methyl ester of formula III, 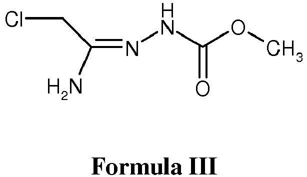 in the presence of potassium carbonate and dimethylsulfoxide to obtain a reaction mixture containing a compound of formula IV; 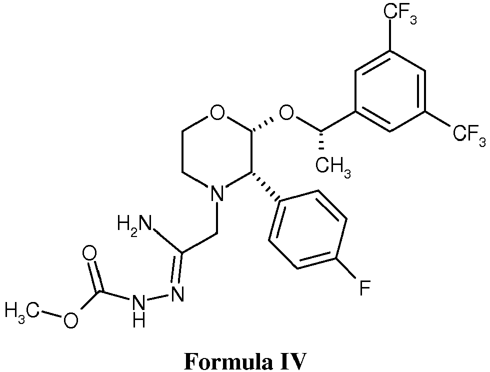
(b) in-situ cyclizing the reaction mixture containing the compound of formula IV to the compound of formula I by heating the resulting reaction mixture in a mixture of dimethylsulfoxide and a polar protic solvent at a temperature of 90-110°C; and
(c) isolating aprepitant of formula I,
wherein the steps (a) and (b) are carried out without isolating intermediate from the reaction mixture.

In an embodiment of the present invention, in step (a) the compound of formula II is reacted with the compound of formula III in the presence of potassium carbonate as a base and dimethylsulfoxide as a solvent at a temperature of 10°C to 30°C for 4 to 5 hours to obtain the reaction mixture containing the compound of formula IV.

In step (b) the reaction mixture obtained containing the compound of formula IV is then treated with a polar protic solvent and the resulting reaction mixture was heated at a temperature of 90-110°C to in-situ cyclize the compound of formula IV to obtain the desired compound of formula I.

In an embodiment of the present invention, in step (b) the polar protic solvent is selected from water, methanol, ethanol, 1-propanol, 2-propanol, 1-butanol or 2-butanol.

In an embodiment of the present invention, in step (b) the polar protic solvent used for the cyclization of compound of formula IV is water.

In an embodiment of the present invention, water is added to the reaction mixture containing the compound of formula IV at a temperature of 10°C to 30°C.

In an embodiment of the present invention, the polar protic solvent is added to the reaction mixture containing the compound of formula IV and heated at a temperature of 90°C to 110°C for 6-10 hours to cyclize the compound of formula IV and obtains the compound of formula I.

In an embodiment of the present invention, the process for preparation of aprepitant of formula I from the 2-(R)-[(1R)-1-[3,5-bis(trifluoromethyl)phenyl]ethoxy]-3-(S)-(4-fluorophenyl)morpholine hydrochloride salt of formula II is carried out in-situ i.e. without isolation of any intermediates at any stage. The process of the present invention involves in-situ cyclization of compound of formula IV, wherein the reaction mixture obtained after the condensation reaction containing the compound of formula IV is cyclized to yield aprepitant without any further work-up processes such as extraction, evaporation, crystallization, etc to isolate the compound of formula IV in the form of solid, foam, viscous liquid, liquid, etc.

In an embodiment of the present invention, the compound of formula II and the compound of formula III condensed in the presence of potassium carbonate and dimethylsulfoxide to yield the reaction mixture containing the compound of formula IV.

The polar protic solvent, such as water was then charged to the reaction mixture and the resulting reaction mixture was heated at a temperature of 90°C to 110°C to cyclize the compound of formula IV to obtain the desired aprepitant of formula I.

In an embodiment of the present invention, the resulting aprepitant of formula I is isolated from the reaction mixture using water as the solvent, wherein water is added to the reaction mixture containing aprepitant.

In an embodiment of the present invention, the reaction mixture obtained after treatment with water containing the compound of formula I is isolated from the reaction mixture by adding an acid to the reaction mixture.

The acid used for the reaction is selected from hydrochloric acid, formic acid, acetic acid, oxalic acid or sulfuric acid. The acid used for the reaction is acetic acid. The obtained reaction mixture heated at a temperature of 65°C to 90°C for 30 minutes and then cooled to a temperature of 20°C to 30°C to precipitate the compound of formula I.

In an embodiment of the present invention, the isolated compound of formula I is further treated with toluene at a temperature of 75°C to 95°C to yield substantially pure aprepitant of formula I, having purity ≥99.5%.

In an embodiment of the present invention, the obtained aprepitant of formula I can be optionally purified using activated charcoal in the presence of methanol at a temperature of 40°C to 70°C to yield pure aprepitant of formula I.

According to the present invention, the starting material 2-(R)-[(1R)-1-[3,5-bis(trifluoromethyl)phenyl]ethoxy]-3-(S)-(4-fluorophenyl)morpholine hydrochloride salt of formula II, potassium carbonate and dimethylsulfoxide are charged to a reaction flask and cooled to a temperature of 10°C to 30°C. To the reaction flask then the compound of formula III is charged and stirred at a temperature of 10°C to 30°C for 4 to 5 hour to obtain the reaction mixture containing the compound of formula IV. To the reaction mixture then charged polar protic solvent, such as water and heated the resulting reaction mixture at a temperature of 90°C to 110°C for 6-10 hours to obtain the compound of formula I. The resulting compound of formula I is isolated from the reaction mixture by adding water to the reaction mixture; followed by adding an acid such as, acetic acid to the reaction mixture, at this stage the pH of the reaction mixture is 6 to 7. The resulting reaction mixture is then heated at a temperature of 65°C to 90°C for 30 minutes and then cooled to a temperature of 20°C to 30°C to precipitate the product, aprepitant of formula I. To the resulting compound of formula I then charged toluene and the reaction mixture is heated at a temperature of 75°C to 95°C for 1 hour and then cooled to a temperature of 20°C to 35°C to precipitate the compound of formula I. Filter the precipitate, wash with toluene and dry under vacuum.

The product, aprepitant of formula I can be optionally purified by charging aprepitant and methanol to the reaction flask and heating the reaction mixture to a temperature of 40°C to 70°C for 1 hour. To the reaction mixture is then added activated charcoal and heating is continued at a temperature of 40°C to 70°C for 1 hour. The reaction mixture is then filtered; and washed with methanol. The filtrate obtained is then cooled and dropwise added water to the filtrate. The filtrate is further cooled to yield aprepitant. The obtained aprepitant is then filtered and dried under vacuum.

The starting material of the process, 2-(R)-[(1R)-1-[3,5-bis(trifluoromethyl)phenyl]ethoxy]-3-(S)-(4-fluorophenyl)morpholine hydrochloride salt, of formula II is a known compound and can be prepared by a person skilled in the art by following process known in the art. For example, the compound of formula II can be prepared by following the process disclosed in the US Patent No. 6600040. The process involves reaction of (2R, 2αR)-4-benzyl-2-[1-[3,5-bis(trifluoromethyl)phenyl]ethoxy]-1,4-oxazin-3-one with 4-fluorophenyl magnesium bromide in the presence of tetrahydrofuran, the resulting reaction mixture was treated with solution of 4-toluenesulfonic acid monohydrate in methanol and 5% Pd/C catalyst under 5 psi of hydrogen pressure to obtain the reaction mixture. The resulting reaction mixture on further work-up process followed by treatment with concentrated hydrochloric acid yields the compound of formula II.

The following examples which fully illustrate the practice of the preferred embodiments of the present invention are intended to be for illustrative purpose only and should not be considered in anyway to limit the scope of the present invention.

### EXAMPLES

### Example 1:

To a 1L round bottom flask charged the compound of formula II (40g), dimethylsulfoxide (100ml) and potassium carbonate (35g) and stirred the reaction mixture for 15 minutes at a temperature of 20°C to 30°C. The reaction mixture is then cooled to a temperature of 10°C to 30°C. To the reaction mixture then charged the compound of formula III (14.9g) and stirred the reaction mixture at a temperature of 10°C to 30°C for 4 to 5 hour. Then charged water (195 ml) to the reaction mixture and heated at a temperature of 90°C to 110°C and maintained for 6-10 hours. To the resulting reaction mixture then added water and stirred for 15 minutes at a temperature of 90°C to 95°C. The reaction mixture is then cooled to the temperature of 40°C to 45°C and then added 50% acetic acid solution in water (∼41ml) to the reaction mixture and stirred for 15 minutes, at this stage pH of the reaction mixture is 6 to 7. The reaction mixture is then heated at a temperature of 65°C to 90°C with stirring for 30 minutes and then cooled to a temperature of 20°C to 30°C and maintained with stirring for 1 hour to obtain the solid. Filter the solid obtained under vacuum and washed with water and suck dry to obtain the solid. To the solid then add toluene (280ml) and heated the reaction mixture at a temperature of 75°C to 95°C for 1 hour. The reaction mixture is then cooled to a temperature of 20°C to 35°C with stirring for 30 minutes to yield the solid. Filter the solid obtained under vacuum and washed with toluene and suck dry to obtain aprepitant. Yield 79% and purity 99.8%.

### Example 2:

To a 1L round bottom flask charged the compound of formula II (13.5Kg), dimethylsulfoxide (34L) and potassium carbonate (11.9kg) and stirred the reaction mixture for 15 minutes at a temperature of 20°C to 30°C. The reaction mixture is then cooled to a temperature of 10°C to 30°C. To the reaction mixture then charged the compound of formula III (5kg) and stirred the reaction mixture at a temperature of 10°C to 30°C for 4 to 5 hour. Then charged water (68L) to the reaction mixture and heated at a temperature of 90°C to 110°C and maintained for 6-10 hours. To the resulting reaction mixture then added water and stirred for 15 minutes at a temperature of 90°C to 95°C. The reaction mixture is then cooled to the temperature of 40°C to 45°C and then added 50% acetic acid solution in water (∼12.5L) to the reaction mixture and stirred for 15 minutes, at this stage pH of the reaction mixture is 6 to 7. The reaction mixture is then heated at a temperature of 65°C to 90°C with stirring for 30 minutes and then cooled to a temperature of 20°C to 30°C and maintained with stirring for 1 hour to obtain the solid. Filter the solid obtained under vacuum and washed with water and suck dry to obtain the solid. To the solid then add toluene (95L) and heated the reaction mixture at a temperature of 75°C to 95°C for 1 hour. The reaction mixture is then cooled to a temperature of 20°C to 35°C with stirring for 30 minutes to yield the solid. Filter the solid obtained under vacuum and washed with toluene and suck dry to obtain aprepitant. Yield 76% and purity 99.7%.

### Example 3:

To a 1L round bottom flask charged the compound of formula II (50g), dimethylsulfoxide (125ml) and potassium carbonate (43.87g) and stirred the reaction mixture for 15 minutes at a temperature of 20°C to 30°C. The reaction mixture is then cooled to a temperature of 10°C to 30°C. To the reaction mixture then charged the compound of formula III (18.35g) and stirred the reaction mixture at a temperature of 10°C to 30°C for 4 to 5 hour. Then charged water (250 ml) to the reaction mixture and heated at a temperature of 90°C to 110°C and maintained for 6-10 hours. To the resulting reaction mixture then added water and stirred for 15 minutes at a temperature of 90°C to 95°C. The reaction mixture is then cooled to the temperature of 40°C to 45°C and then added 50% acetic acid solution in water (∼53ml) to the reaction mixture and stirred for 15 minutes, at this stage pH of the reaction mixture is 6 to 7. The reaction mixture is then heated at a temperature of 65°C to 90°C with stirring for 30 minutes and then cooled to a temperature of 20°C to 30°C and maintained with stirring for 1 hour to obtain the solid. Filter the solid obtained under vacuum and washed with water and suck dry to obtain the solid. To the solid then add toluene (350ml) and heated the reaction mixture at a temperature of 75°C to 95°C for 1 hour. The reaction mixture is then cooled to a temperature of 20°C to 35°C with stirring for 30 minutes to yield the solid. Filter the solid obtained under vacuum and washed with toluene and suck dry to obtain aprepitant. Yield 79% and purity 99.5%.

To the aprepitant charged methanol (500 ml) and heated at a temperature of 40°C to 70°C and stirred for 1 hour. Then charged activated charcoal (2.5 g) and heated at a temperature of 40°C to 70°C with stirring for 1 hour. Filter the charcoal through hyflow bed and wash with methanol. The filtrate obtained is then cooled and dropwise added water to the filtrate. The filtrate further cooled to precipitate the solid. Filter the solid obtained under vacuum and wash with solution of methanol in water. Dry the solid under vacuum to yield aprepitant.

**Analytical method for analysis**

| | |
|---|---|
| HPLC column | : L1 column |
| Detector | : 210 nm |
| Mobile phase A | : Trifluroacetic acid (TFA), water and triethylamine |
| Mobile Phase B | : Acetonitrile |
| Injection volume | : 20µL |
| Column temperature | : 30°C |
| Flow rate | : 1.0 mL/minutes |
| Run time | : 50 minutes |
| Gradient : | |

| | | |
|---|---|---|
| **Time** | **%A** | **%B** |
| 0 | 52 | 48 |
| 20 | 52 | 48 |
| 40 | 10 | 90 |
| 45 | 10 | 90 |
| 45.1 | 52 | 48 |
| 50 | 52 | 48 |

## Claims

1. A process for the preparation of 5-[[(2R,3S)-2-[(1R)-1-[3,5-bis(trifluoromethyl)phenyl]ethoxy]-3-(4-fluorophenyl)-4-morpholinyl]methyl]-1,2-dihydro-3H-1,2,4-triazol-3-one (Aprepitant) of formula I, 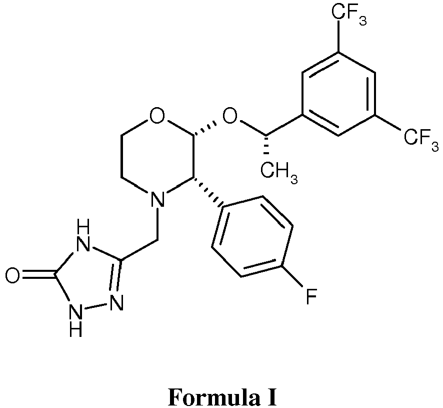 comprising the steps of:
(a) condensing 2-(R)-[(1R)-1-[3,5-bis(trifluoromethyl)phenyl]ethoxy]-3-(S)-(4-fluorophenyl)morpholine hydrochloride salt of formula II 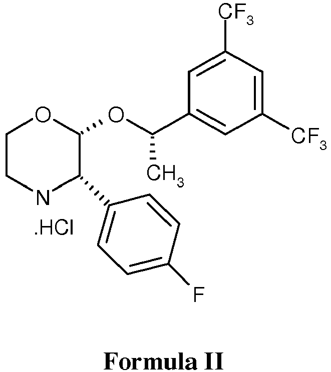 with 2-(2-chloro-l-iminoethyl)hydrazinecarboxylic acid methyl ester of formula III 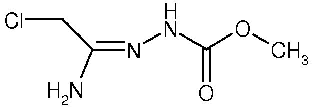 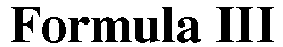 in the presence of potassium carbonate and dimethylsulfoxide to obtain a reaction mixture containing 2-[2-[(2R,3S)-2-[(1R)-1-[3,5-bis(trifluoromethyl)phenyl]ethoxy]-3-(4-fluorophenyl)-4-morpholinyl]-1-iminoethyl]hydrazinecarboxylic acid methyl ester of formula IV; 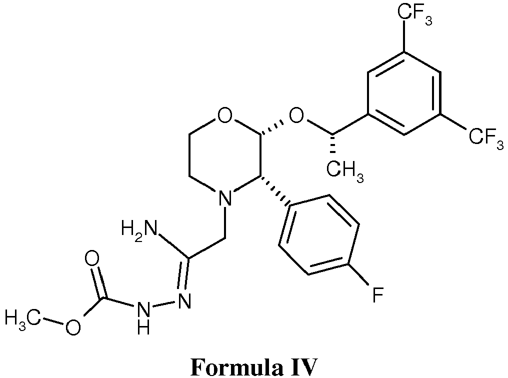
(b) in-situ cyclizing the reaction mixture containing compound of formula IV to the compound of formula I by heating said reaction mixture in a mixture of dimethylsulfoxide and a polar protic solvent at a temperature of 90-110°C and
(c) isolating aprepitant of formula I, wherein the steps (a) and (b) are carried out without isolating intermediates from the reaction mixture.

2. The process as claimed in claim 1, wherein in the step (b) the polar protic solvent is added to the reaction mixture before heating the reaction mixture.

3. The process as claimed in claim 1, wherein in the step (c) the compound of formula I is isolated using water.

4. The process as claimed in claim 3, further comprises use of an acid to isolate the compound of formula I.

5. The process as claimed in claims 1, 3 or 4, wherein the isolated compound of formula I is treated with toluene to yield the compound of formula I having purity ≥ 99.5%.

6. The process as claimed in claim 1 or 2, wherein said polar protic solvent is selected from water, methanol, ethanol, 1-propanol, 2-propanol, 1-butanol or 2-butanol.

7. The process as claimed in claim 6, wherein said polar protic solvent is water.

8. The process as claimed in claim 4, wherein said acid is selected from hydrochloric acid, formic acid, acetic acid, oxalic acid or sulfuric acid.

9. The process as claimed in claim 8, wherein said acid is acetic acid.

## Patentansprüche

1. Verfahren zur Herstellung von 5-[[(2R,3S)-2-[(1R)-1-[3,5-Bis(trifluormethyl)-phenyl]-ethoxy]-3-(4-fluorophenyl)-4-morpholinyl]methyl]1,2-dihydro-3H-1,2,4-triazol-3-on (Aprepitant) der Formel I, 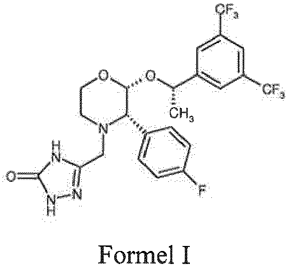 welches die Schritt umfasst:
(a) Kondensieren des Hydrochlorid-Salzes von 2-(R)-[(1R)-1-[3,5-Bis(trifluormethyl)-phenyl]ethoxy]-3-(S)-(4-fluorphenyl)-morpholin der Formel II 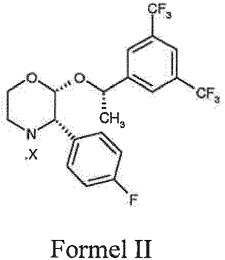 mit 2-(2-Chlor-1-iminoethyl)hydrazincarbonsäuremethylester der Formel III 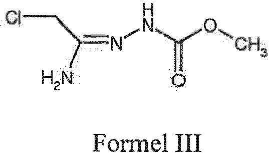 in Anwesenheit von Kaliumcarbonat und Dimethylsulfoxid, um ein Reaktionsgemisch zu erhalten, das 2-[2-[(2R,3S)-2-[(1R)-1-[3,5-Bis(trifluormethyl)phenyl]ethoxy]-3-(4-fluorphenyl)-4-morpholinyl]-1-iminoethyl]hydrazincarbonsäuremethylester der Formel IV enthält; 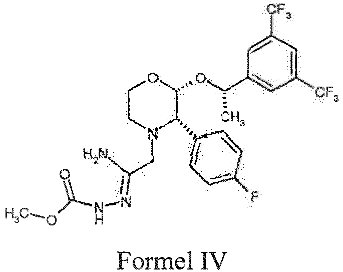
(b) in-situ Zyklisieren des Reaktionsgemisches, das die Verbindung der Formel IV enthält, zur Verbindung der Formel I durch Erhitzen des Reaktionsgemisches in einem Gemisch von Dimethylsulfoxid und einem polaren protischen Lösungsmittel bei einer Temperatur von 90-110 °C, und
(c) Isolieren von Aprepitant der Formel I,
wobei die Schritte (a) und (b) ohne Isolierung der Zwischenprodukte aus dem Reaktionsgemisch durchgeführt werden.

2. Verfahren nach Anspruch 1, wobei in Schritt (b) das polare protische Lösungsmittel vor Erhitzen des Reaktionsgemisches zu dem Reaktionsgemisch zugesetzt wird.

3. Verfahren nach Anspruch 1, wobei in Schritt (c) die Verbindung der Formel 1 unter Verwendung von Wasser isoliert wird.

4. Verfahren nach Anspruch 3, welches weiter die Verwendung einer Säure umfasst, um die Verbindung der Formel I zu isolieren.

5. Verfahren nach Anspruch 1, 3 oder 4, wobei die isolierte Verbindung der Formel I mit Toluol umgesetzt wird, um die Verbindung der Formel I mit einer Reinheit von ≥ 99,5 % zu erhalten.

6. Verfahren nach Anspruch 1 oder 2, wobei das polare protische Lösungsmittel ausgewählt ist aus der Gruppe bestehend aus Wasser, Methanol, Ethanol, 1-Propanol, 2-Propanol, 1-Butanol oder 2-Butanol.

7. Verfahren nach Anspruch 6, wobei das polare protische Lösungsmittel Wasser ist.

8. Verfahren nach Anspruch 4, wobei die Säure ausgewählt ist unter Salzsäure, Ameisensäure, Essigsäure, Oxalsäure oder Schwefelsäure.

9. Verfahren nach Anspruch 8, wobei die Säure Essigsäure ist.

## Revendications

1. Procédé de préparation de la 5-[[(2R,3S)-2-[(1R)-1-[3,5-bis(trifluorométhyl)phényl]éthoxy]-3-(4-fluorophényl)-4-morpholinyl]méthyl]-1,2-dihydro-3H-1,2,4-triazol-3-one (Aprepitant) de formule I 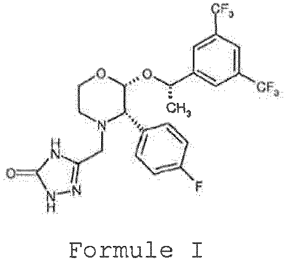 comprenant les étapes de :
(a) condensation d'un sel chlorhydrate de 2-(R)-[(1R)-1-[3,5-bis(trifluorométhyl)phényl]éthoxy]-3-(S)-(4-fluorophényl)morpholinyle de formule II 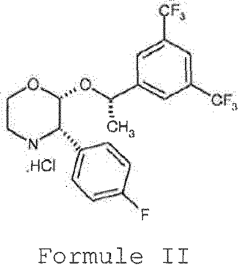 avec de l'ester méthylique de l'acide 2-(2-chloro-1-iminoéthyl)hydrazine-carboxylique de formule III 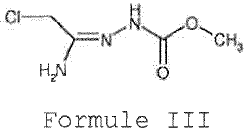 en présence de carbonate de potassium et de diméthylsulfoxyde pour obtenir un mélange réactionnel contenant l'ester méthylique de l'acide 2-[2-[(2R,3S)-2-[(1R)-1-[3,5-bis(trifluorométhyl)phényl]éthoxy]-3-(4-fluorophényl)-4-morpholinyl]-1-iminoéthyl)hydrazine-carboxylique de formule IV 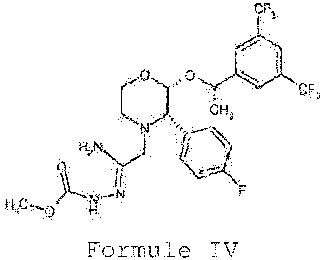
(b) cyclisation in situ du mélange réactionnel contenant le composé de formule IV pour donner le composé de formule I par chauffage dudit mélange réactionnel dans un mélange de diméthylsulfoxyde et d'un solvant protique polaire à une température de 90 à 110°C, et
(c) isolement de l'aprepitant de formule I,
dans lequel les étapes (a) et (b) sont réalisées sans isolement des intermédiaires du mélange réactionnel.

2. Procédé selon la revendication 1, dans lequel dans l'étape (b) le solvant protique polaire est ajouté au mélange réactionnel avant le chauffage du mélange réactionnel.

3. Procédé selon la revendication 1, dans lequel dans l'étape (c) le composé de formule I est isolé à l'aide d'eau.

4. Procédé selon la revendication 3, qui comprend en outre l'utilisation d'un acide pour isoler le composé de formule I.

5. Procédé selon les revendications 1, 3 ou 4, dans lequel le composé de formule I isolé est traité avec du toluène pour produire le composé de formule I ayant une pureté ≥ 99,5 %.

6. Procédé selon la revendication 1 ou 2, dans lequel ledit solvant protique polaire est choisi parmi l'eau, le méthanol, l'éthanol, le 1-propanol, le 2-propanol, le 1-butanol ou le 2-butanol.

7. Procédé selon la revendication 6, dans lequel ledit solvant protique polaire est l'eau.

8. Procédé selon la revendication 4, dans lequel ledit acide est choisi parmi l'acide chlorhydrique, l'acide formique, l'acide acétique, l'acide oxalique ou l'acide sulfurique.

9. Procédé selon la revendication 8, dans lequel ledit acide est l'acide acétique.
